# EUROPEAN PATENT APPLICATION

(11) **EP 4 789 618 A1**
(43) Date of publication of application: **12.08.2026**
(21) Application number: 25156642.8
(22) Date of filing: 07.02.2025
(51) Int. Cl.: A61B 6/10, G21F 3/00, A61N 5/10

(54) **RADIATION-PROTECTIVE COVERING COMPRISING RADIATION-TRANSPARENT PORTION**

(71) Applicant: Texray AB, 412 92 Göteborg (SE)
(72) Inventor: APELL, Petra, 439 37 ONSALA (SE); GELLERSTEDT, Fredrik, 413 09 GÖTEBORG (SE); STÅHL, Shadi, 431 36 MÖLNDAL (SE)
(74) Representative: Öberg & Sjöberg AB

(57) **Abstract**

A radiation-protective covering (10) comprising: a radiation-protective portion (14) comprising a flexible radiation-protective material, and a radiation-transparent portion (34) comprising a flexible radiation-transparent material, and a flexible radiation-protective patch (36), comprising a flexible radiation-protective material, configured to: cover the radiation-transparent portion in a first position, and uncover at least part of the radiation-transparent portion in a second position. A use of the radiation-protective covering (10) for protecting clinical staff (6) from radiation (8) during a medical intervention comprising the use of radiation is also disclosed.

## Description

### Technical field

The technology proposed herein relates to radiation-protective coverings, and particularly to X-ray protective coverings comprising a radiation-transparent portion.

### Background

Ionizing radiation, such as X-ray, is commonly used in radiologically guided interventions, such as cardiology, or Interventional Radiology (IR). The clinical staff is exposed to the radiation during the procedures. Current standards and practices are based on the premise that any radiation dose may result in unfavorable health effects and the development of radiation-induced diseases including glioblastoma. Reducing the radiation exposure of the clinical staff is therefore important.

The radiation can be direct or scattered. In particular, radiation incident on a patient or a part of a patient will scatter, both at the point where the radiation impinges on the patient but also throughout the body of the patient. Further scattering occurs as the radiation impinges on the operating table.

During the radiologically guided intervention, or procedure, the patient is typically placed on an operating table. A source of radiation is provided above or underneath the operating table. The source of radiation may be positioned in various angular projections in relation to the patient. One or more radiation-protective drapes or coverings may be placed on the patient to prevent a major part of the radiation scattered in the body of the patient from irradiating out of the patient to reach the clinical staff. During the radiologically guided intervention the clinical staff typically stand along the left long side of the operating table for access to the interventional vascular entry points such as femoralis or radialis.

WO 2023/163643 discloses a radiation protection shielding system comprising a radiation protection drape sized to at least partially cover the patient from a proximity of a radiated area of the patient to the feet of the patient. As such, the radiation protection drape will prevent a major part of the radiation scattered in the body of the patient from irradiating out of the patient to reach the clinical staff.

For clinical procedures in the head of the patient, there is a need for a radiation-protective covering that provides suitable protection against scattering yet is flexible to use.

There is also a need for a radiation-protective covering flexible to use for clinical procedures involving passing surgical instruments or medical devices through major passageways of the body, such as major blood vessels.

### Object of the proposed technology

The proposed technology aims at providing an alternative to other radiation-protective coverings that is better suited to protect the clinical staff during procedures in the head of a patient yet provides more flexibility for use.

It is a further object of the proposed technology to provide a radiation-protective covering that is easy to apply on the patient yet provides ease of access to the patient when needed during a procedure in the head of the patient.

It is a further object of the proposed technology to provide a covering suitable for use in clinical procedures involving passing surgical instruments or medical devices through major passageways of the body, such as major blood vessels.

### Summary

A first aspect of the proposed technology concerns a radiation-protective covering comprising:
- a radiation-protective portion comprising a flexible radiation-protective material, and
- a radiation-transparent portion comprising a flexible radiation-transparent material, and
- a flexible radiation-protective patch, comprising a flexible radiation-protective material, configured to:
   ∘ cover the radiation-transparent portion in a first position, and
   ∘ uncover at least part of the radiation-transparent portion (34) in a second position.

The present inventors have thus found that when performing clinical procedures in the head of a patient, increased protection and flexibility can be obtained by using a radiation-protective covering according to the first aspect of the proposed technology placed over the torso of the patient. Specifically, due to the position of the clinical staff at the side of the patient, using a femoral access as entry to the procedure, it has been found that a major effect on radiation protection from scattered radiation can be obtained by covering the torso whereas extremities as legs and arms may play less role as being further away from the radiated area. Thus, a radiation-protective covering placed over the torso may provide sufficient protection.

More importantly and compared to other radiation-protective coverings having apertures in the torso portion, e.g. for allowing x-ray imaging of the heart or nearby organs, the flexible radiation-protective patch provides for fully preventing any scattered radiation from irradiating the clinical staff with the flexible radiation-protective patch in the first position.

Yet, and most importantly, the flexible radiation-protective patch can be put into the second position if desired, for example if there is a need to image the position of a surgical instrument or medical device, such as a catheter or other equipment being manoeuvred through a major passageway of the body, such as the aorta and/or the major blood vessels towards a position in the head of the patient. This is because imaging using radiation is possible through any part of the radiation-transparent portion of the radiation-protective covering.

Another example where increased protection and flexibility can be obtained by using a radiation-protective covering according to the first aspect of the proposed technology when other passageways are used. For example, the covering may be placed over a leg of the patient whereby a stent may be placed below the knee of the patient by using the femoral access to guide the catheter, stent and/or other equipment through the iliac vessel and/or other major blood vessels in the upper leg. When needed the flexible radiation-protective patch may be positioned in the second position to allow imaging the iliac region to inspect the manoeuvring of the guide wires during the intervention. When such imaging is not needed, the flexible radiation-protective patch is positioned in the first position to protect from radiation emanating through the radiation-transparent portion.

The radiation-protective covering may preferably be for covering at least a part of a patient during a clinical procedure involving radiation. Expressed differently, the radiation-protective covering has an intended position covering at least part of a patient, in particular a patient in a supine or prone position. The part of the patient preferably comprises a major passageway, such as a major blood vessel, where radiation imaging examination may be desired to be conducted. The part of the patient may thus preferably comprise the torso, whereby the aorta may be imaged through the radiation-transparent portion, or an upper leg of the patient, whereby the iliac region may be imaged.

The radiation-protective covering may have a first dimension, preferably a length dimension, which may be defined along the length of a patient when the radiation-protective covering is in the intended position, which may be up to 150 cm, preferably up to 120 cm, more preferably up to 100 cm. The first dimension may be at least 10 cm, preferably at least 20 cm, more preferable at least 30 cm. The first dimension may thus be in the range of 10 to 150 cm, preferably 20 to 120 cm, more preferably 30 to 100 cm.

The radiation-protective covering may have a second dimension, preferably a width dimension, which may be defined along the width of a patient when the radiation-protective covering is in the intended position. The second dimension is perpendicular to the first dimension. The second dimension may be up to 120 cm, preferably up to 100 cm, more preferably up to 90 cm. The second dimension may be at least 20 cm, preferably at least 40 cm, more preferably at least 60 cm. The second dimension may thus be in the range of 20 to 120 cm, preferably 40 to 100 cm, more preferably 60 to 90 cm.

The radiation-protective covering may have a third dimension, preferably a thickness dimension, which is perpendicular to the first and second dimensions, and which may be at least 1 mm, preferably at least 2 mm, preferably at least 3 mm. The third dimension may be up to 20 mm, preferably up to 15 mm, more preferably up to 10 mm. The third dimension may thus be in the range of 1 to 20 mm, preferably 2 to 15 mm, more preferably 3 to 10 mm.

The radiation-protective covering may preferably be rectangular. The radiation-protective covering may alternatively have other shapes such as an L-shape or rectangular hexagon, a polygonal shape such as a pentagonal shape or an octagonal shape, a circular or elliptical shape, or a trapezoidal shape. For such shapes, the length of the radiation-protective covering, extends between the point on the edge of the radiation-protective covering that is closest to the head of a patient when the radiation-protective covering is in the intended position, and the opposite point on the edge that is positioned closest to the feet of the patient when the radiation-protective covering is in the intended position. Further, the width extends between the points on the edge of the radiation-protective covering that are furthest from each other along a line perpendicular to the length.

It is specified that the covering is radiation-protective. Expressed differently, the covering protects against, or attenuates, ionizing radiation. Expressed differently, the covering may be an X-ray protective covering. The covering may comprise a radiation attenuating material, in particular an X-ray attenuating material. The covering may be arranged or configured to attenuate X-ray radiation from a radiation source having a tube voltage of at least with energies of at least 40 kV, preferably at least 60 kV, and at the most 150 kV, preferably at the most 110 kV. For example, the covering may be arranged or configured to attenuate X-ray radiation from a radiation source having a tube voltage with energies at least in the range of 40-150 kV, such as 60-110 kV. The covering may be arranged or configured to provide at least 0.05, mm, preferably at least 0.10 mm, more preferably at least 0.25 mm, most preferably at least 0.50 mm, and at the most 1.00 mm, preferably at the most 0.75 mm, more preferably at the most 0.50 mm Pb equivalence at any tube voltage in the range 60-110 kV. For example, the covering may be arranged or configured to provide 0.05 to 1.00 mm, preferably 0.10 to 1.00 mm, more preferably 0.25 to 1.00 mm, such as 0.25 to 0.75 mm or 0.30 to 0.60 mm, in particular at least 0.50 mm, Pb equivalence, at any tube voltage in the range 60-110 kV. Pb equivalence is to be measured according to Inverse Broad Beam Geometry as described in IEC 61331-1:2014. The covering may be non-transparent or non-translucent to optical, or visible, light.

The radiation-protective portion and the radiation-transparent portion may be arranged with the latter within the former, i.e. with the radiation-transparent portion being provided as a portion within the radiation-protective portion. This may for example be implemented by providing an aperture or peripheral cutout in the radiation-protective portion and providing the radiation-transparent portion within, or covering, the aperture of or peripheral cutout.

Alternatively, the radiation-protective portion and the radiation-transparent portion may be arranged with one adjoining the other, i.e. with the radiation-protective portion arranged side by side, with or without overlap, with the radiation-transparent portion. The radiation-protective portion may make up at least 30%, preferably at least 40%, more preferably at least 50%, most preferably at least 60% of the combined area of the radiation-protective portion and the radiation-transparent portion.

The radiation-protective portion may be quadrilateral or rectangular, or have any of the shapes described above for the radiation-protective covering.

The radiation-protective portion may have a length, in the same direction as the length of the radiation-protective covering described above, of up to 150 cm, preferably up to 120 cm, more preferably up to 100 cm. The length may be at least 10 cm, preferably at least 20 cm, more preferable at least 30 cm. The first dimension may thus be in the range of 10 to 150 cm, preferably 20 to 120 cm, more preferably 30 to 100 cm. The length may thus be in the range of 10 to 150 cm, preferably 20 to 120 cm, more preferably 30 to 100 cm.

The radiation-protective portion may have a width, in the same direction as the width of the radiation-protective covering described above, of up to 120 cm, preferably up to 100 cm, more preferably up to 90 cm. The width may be at least 20 cm, preferably at least 40 cm, more preferably at least 70 cm. The width may thus be in the range of 20 to 120 cm, preferably 40 to 100 cm, more preferably 70 to 90 cm.

The radiation-protective portion may have a thickness which may be at least 1 mm, preferably at least 2 mm, more preferably at least 3 mm. The third dimension may be up to 20 mm, preferably up to 15 mm, more preferably up to 10 mm. The thickness may thus be in the range of 1 to 20 mm, preferably 2 to 15 mm, more preferably 3 to 10 mm.

The radiation-transparent portion may be any of quadrilateral, rectangular, polygonal, ellipsoid or circular. Typically, the area of the radiation-transparent portion is less than the area of the radiation-protective portion.

The radiation-transparent portion may have a length, in the same direction as the length of the radiation-protective covering described above, of up to 60 cm, preferably up to 40 cm, more preferably up to 30 cm. The length may be at least 5 cm, preferably at least 10 cm, more preferably at least 20 cm. The length may thus be in the range of 5 to 60 cm, preferably 10 to 40 cm, more preferably 20 to 30 cm.

The radiation-transparent portion may have a width, in the same direction as the width of the radiation-protective covering described above, of up to 50 cm, preferably up to 40 cm, more preferably up to 20 cm. The width may be at least 5 cm, preferably at least 10 cm, more preferable at least 15 cm. The width may thus be in the range of 5 to 50 cm, preferably as 10 to 40 cm, more preferably 15 to 20 cm.

The radiation-transparent portion may have a thickness which may be at least 1 mm, preferably at least 2 mm, more preferably at least 3 mm. The thickness may be up to 20 mm, preferably up to 15 mm, more preferably up to 10 mm. The thickness may thus be in the range of 1 to 20 mm, preferably 2 to 15 mm, more preferably 3 to 10 mm.

It is understood that the radiation-transparent portion is made up of a material, i.e. the second-radiation material is not merely a fenestration or cutout, or absence of material, in the radiation-protective portion.

A fenestration or aperture may be provided in the radiation-transparent portion. The fenestration or aperture as passageway for medical equipment. The fenestration or aperture may have a maximum diameter of at least 5 cm, preferably at least 10 cm, and at the most 20 cm, preferably at the most 15 cm. Alternatively, the fenestration or aperture may be sized so as to make up to 95% of the area of the radiation-transparent portion, preferably up to 90%, such as 50%-95%, preferably 60% to 90%, of the area of the radiation-transparent portion. In both cases the radiation-transparent portion forms a border around the fenestration or aperture. The border may have a width of 0.5 cm to 5 cm, preferably 1 cm to 4 cm.

It is specified that the radiation-protective portion comprises a flexible radiation-protective material.

Expressed differently, the flexible radiation-protective material is capable of flexing, or deforming, when subjected to force, such as force applied on the flexible radiation-protective material. The flexible radiation-protective material is thus pliable. The flexible radiation-protective material may be at least partially elastic, or resilient, whereby the flexible radiation-protective material essentially, or substantially, resumes an initial shape and configuration once a force deforming it is no longer applied.

The flexible radiation-protective material may comprise, or consist of, a permeable fabric, or fibrous material. The permeable fabric may comprise filaments. The filaments may be arranged in a regular pattern. The filaments may extend in a uniform direction. Each filament may be of a solid composition that comprises a carrier substance and a radiopaque substance. For example, the solid composition may be a polymeric composite material. The filaments may be arranged in a regular pattern. It is understood that each filament may be a monofilament. It is further understood that the carrier substance and the radiopaque substance may be mixed, and that the radiopaque substance may be evenly distributed within the carrier substance. The carrier substance may comprise at least one polymer and the radiopaque substance may comprise at least one metal. For example, the polymer may be a thermoplastic polymer such a polyvinyl chloride or a polyolefin and the metals may, as a powder, be as an element, oxide or alloy of for example Lead, Barium, Antimony, Bismuth, or Tungsten (Wolfram), and any combination thereof.

The fabric may be a woven fabric with the filaments forming the weft of the fabric. It is understood that the fabric has a warp, for example of polyester. More specifically, the first material may be any of the radiation-protective material described in WO2014163574A1. The fabric contributes to higher longevity of the radiation-protective covering.

Alternatively, the flexible radiation-protective material may be, or comprise, an impermeable sheet. The impermeable sheet may be of a solid composition that comprises a carrier substance and a radiopaque substance. For example, the solid composition may be a polymeric composite material. It is further understood that the carrier substance and the radiopaque substance may be mixed, and that the radiopaque substance may be evenly distributed within the carrier substance. The carrier substance may comprise at least one polymer and the radiopaque substance may comprise at least one metal.

It is understood that the flexible radiation-protective material may be composed of several layers, for example two layers of fabric or two impermeable sheets. As specified above for the radiation-protective covering, the flexible radiation-protective material may have at least 0.05, mm, preferably at least 0.10 mm, more preferably at least 0.25 mm, most preferably at least 0.50 mm, and at the most 1.00 mm, preferably at the most 0.75 mm, more preferably at the most 0.50 mm Pb equivalence at any tube voltage in the range 60-110 kV. For example, the covering may be arranged or configured to provide 0.05 to 1.00 mm, preferably 0.10 to 1.00 mm, more preferably 0.25 to 1.00 mm, such as 0.25 to 0.75 mm or 0.30 to 0.60 mm, most preferably at least 0.50 mm Pb equivalence at any tube voltage in the range 60-110 kV.

The flexible radiation-protective material may have a thickness of at least 0,5 mm, preferably at least 1 mm. The flexible radiation-protective material may have a thickness of at the most 10 mm, preferably at the most 5 mm, such as 0.5 to 10 mm, preferably 1 to 5 mm or 2 to 5 mm.

It is specified that the radiation-transparent portion comprises a flexible radiation-transparent material. The flexible radiation-transparent material is flexible as described above for the flexible radiation-protective material. The flexible radiation-transparent material is a material being less radiation-protective than the flexible radiation-protective material. The flexible radiation-transparent material preferably has less than 0.05, mm, more preferably less than 0.01 mm, such as less than 0.001 mm, most preferably less than 1 µm Pb equivalence at any tube voltage in the range 60-110 kV. Most preferably the flexible radiation-transparent material has substantially 0 Pb equivalence at any tube voltage in the range 60-110 kV. Expressed differently, the flexible radiation-transparent material provides no protection from radiation at any tube voltage in the range 60-110 kV.

Suitable flexible radiation-transparent materials include fabrics, e.g. woven or non-woven, fabrics made from natural fibers such as cotton or wool or made from synthetic fibres such as nylon or polyester, mesh, e.g. mesh made from fabrics or impermeable sheets made from natural or synthetic polymers, nets made from natural or synthetic fibers, and impermeable sheets made from natural or synthetic polymer. Impermeable sheets may for example comprise or consist of plastic sheets, such as sheets made from polyolefins, polyesters or polyvinyl chloride (PVC). It is specified that the material may be a combination of any material mentioned above.

Preferably the flexible radiation-transparent material is a woven or non-woven fabric made from natural fibers such as cotton or wool or made from synthetic fibres such as nylon or polyester. The flexible radiation-transparent materials may more preferably be made from a woven fabric of natural fibers such as cotton or synthetic fibres such as nylon or polyester, or from a mixture of natural fibres and synthetic fibres. Generally, materials used for drapes, coverings and blankets in healthcare may be used as the flexible radiation-transparent material.

The flexible radiation-transparent materials may further be stretchable or elastic.

The flexible radiation-transparent materials may further be optically transparent.

The flexible radiation-protective material of the flexible radiation-protective patch may be as defined above for the radiation-protective portion.

The flexible radiation-protective patch preferably has at least the same dimensions and shape as the radiation-transparent portion so as to be able to completely cover the radiation-transparent portion in the first position. The flexible radiation-protective patch may be divided into two patch portions which in the first position may adjoin each other along opposite edges, and in the second position may be folded away from each other.

It is specified that the flexible radiation-protective patch is configured to cover the radiation-transparent portion in the first position. Expressed differently, the flexible radiation-protective patch is configured to increase the radiation-protection of the radiation-transparent portion in the first position, i.e. compared to when in the second position, where the flexible radiation-protective patch is positioned furthest away from its first position thus revealing/exposing the radiation-transparent portion. Further expressed differently, the flexible radiation-protective patch is configured to increase the radiation-protection of the radiation-protective covering in the first position, i.e. compared to when in the second position.

It is specified that the flexible radiation-protective patch is configured to uncover at least part of the radiation-transparent portion in the second position. Expressed differently, the flexible radiation-protective patch is configured to decrease the radiation-protection of the radiation-transparent portion in the second position, i.e. compared to when in the first position. Further expressed differently, the flexible radiation-protective patch is configured to decrease the radiation-protection of the radiation-protective covering in the second position, i.e. compared to when in the first position.

Further expressed differently, the flexible radiation-protective patch is configured to provide for the passage of radiation through the radiation protective covering in its second position, and to prevent or hinder the passage of radiation through the radiation protective covering in the first position.

Preferably the flexible radiation-protective patch is configured to uncover all of the of the radiation-transparent portion in the second position.

The flexible radiation-protective patch may be configured to be removed from the radiation-protective covering in the second position.

This is advantageous for higher levels of protection where the thickness, stiffness and/or weight of flexible radiation-protective material in the flexible radiation-protective patch may make it more difficult to fold the flexible radiation-protective patch from the first position to the second position. Further, it may be useful when performing clinical procedures where the flexible radiation-protective patch is to be in the second position for the majority of time.

Preferably the flexible radiation-protective patch is configured to be removably attachable to the radiation-protective covering.

This is advantageous in that it provides for easily replacing the flexible radiation-protective patch when or if it becomes worn to a higher extent than the remainder of the radiation-protective covering.

The flexible radiation-protective patch may be configured to be removably attachable to the radiation-protective covering by the use of hook and loop fasteners, magnets, snap fasteners, a zipper, or the like, provided between the flexible radiation-protective patch and the radiation-protective covering.

Alternatively, the flexible radiation-protective patch is permanently attached to the radiation-protective covering, preferably wherein the flexible radiation-protective patch is integrally formed with the radiation-protective portion.

This is advantageous in that it simplifies the work of the clinical staff since the flexible radiation-protective patch is always present, in the first position or the second position, on the radiation-protective covering.

The flexible radiation-protective patch may be permanently attached to the radiation-protective covering by any of stitching, welding, and adhesive, or by being formed integrally with the radiation-protective covering such as by being formed integrally with the first or second flexible radiation-protective sheets.

This is advantageous in that it leads to a simple construction of the radiation-protective covering.

Preferably the flexible radiation-protective patch is configured to be folded from the first position to the second position.

Folding provides an easy way of moving the flexible radiation-protective patch from the first position to the second position. Folding may preferably be used when the flexible radiation-protective patch is permanently attached to the radiation-protective covering or when the flexible radiation-protective patch is integrally formed with one of the first and second flexible radiation-protective sheets. The flexible radiation-protective patch may be configured to be folded from the first position to a stable second position, e.g. without the need for fastening the flexible radiation-protective patch to the radiation-protective covering, using gravity to remain in a second position. The flexible radiation-protective patch may be configured to be folded from the first position to a second position by being attached to the radiation-protective covering at one or more points arranged in a line. This may be implemented using for example a line of snap fasteners or hook and loop for a removable attachment, or by a line of spot welds, stitching, or rivets for a permanent attachment. The flexible radiation-protective patch may be configured to be folded from the first position to the second position by being attached to the radiation-protective covering along a continuous line. This may be implemented using for example a line of stitching, a continuous weld or continuous string of adhesive.

Preferably the radiation-protective covering is shaped as a rectangular hexagon or L-shape.

This is advantageous because the need for radiation protection for the clinical staff is often higher on one side of the patient and/or operating table than on the other. Forming the radiation-protective covering as a rectangular hexagon or L-shape thus generally provides one arm or part of the rectangular hexagon or L-shape position able over the torso of the patient, whereby the other, perpendicular arm or part of the rectangular hexagon or L-shape then will extend along the side of the patient, e.g. so as to cover the side of the patient and extend down along the side of the patient, covering the side and one arm of the patient to thereby provide increased protection from radiation scattered in the patient to clinical staff positioned at that side of the patient. The width of the perpendicular arm or part of the rectangular hexagon or L-shape extending along the side of the patient may typically be at least 10 cm.

Any side of the radiation-protective covering may be further shaped, e.g. rounded or curved, to follow the shape of the patient.

Preferably the radiation-transparent portion is provided within one of the arms of the rectangular hexagon or L-shape.

This is advantageous in that it assists positioning the radiation-transparent portion within the part of the radiation-protective covering that overlies the torso, and in particular the aorta, during use of the radiation-protective covering, or within the part of the radiation-protective covering that overlies a leg, in particular the iliac vessel, during use of the radiation-protective covering.

Preferably the radiation-protective portion comprises:
- a first flexible radiation-protective sheet, and
- a second flexible radiation-protective sheet,

wherein the radiation-transparent portion comprises:
   - a flexible radiation-transparent sheet,
and wherein the first and second flexible radiation-protective sheets are spaced apart from each other by, and attached to, the flexible radiation-transparent sheet.

This is advantageous in that it provides a large radiation-transparent portion.

The flexible radiation-protective sheets comprise flexible radiation-protective material as described above and are flexible as described above for the flexible radiation-protective material.

The flexible radiation-protective sheet comprises flexible radiation-transparent material as described above and is flexible as described above for the flexible radiation-transparent material.

As the first and second flexible radiation-protective sheets are attached to the flexible radiation-protective sheet, the radiation-protective covering is convenient to place on the patient and there is no risk of the first and second flexible radiation-protective sheets sliding further apart or sliding off the patient as would be the case if the first and second flexible radiation-protective sheets were not attached to the flexible radiation-protective sheet. In other words, the flexible radiation-transparent sheet serves to position and maintain the position of the first and second flexible radiation-protective sheets relative to each other.

The first flexible radiation-protective sheet, as well as the second flexible radiation-protective sheet, may be generally rectangular. Expressed differently, the first and flexible radiation-protective sheets may have opposite upper and lower edges, e.g. opposite first and second short sides, and opposite left and right edge, i.e. opposite first and second long sides.

The upper edge or second short side of the first and second flexible radiation-protective sheets may generally be positioned closer to the head of a patient at least partially covered by the radiation-protective covering than the lower edge or first short side, which generally is positioned closer the waist of a patient at least partially covered by the radiation-protective covering.

The first and second flexible radiation-protective sheets, may generally have a length, from the lower edge to the upper edge, i.e. from first short side to second short side, which may be up to 150 cm, preferably up to 120 cm, more preferably up to 100 cm. The length dimension may be at least 10 cm, preferably at least 20 cm, more preferable at least 30 cm. The length dimension may thus be in the range of 10 to 150 cm, such as 20 to 120 cm, preferably 30 to 100 cm.

The first and second radiation-protective sheets may generally have a width, from the left edge to the right edge, from a first long side to a second long side, which may be up to 60 cm, preferably up to 50 cm, more preferably up to 45 cm. The width may be at least 10 cm, preferably at least 20 cm, more preferable at least 35 cm. The width may thus be in the range of 10 to 60 cm, preferably 20 to 50 cm, more preferably 35 to 45 cm.

The first and second radiation-protective sheets may generally have a thickness, which may be at least 1 mm, preferably at least 2 mm, more preferably at least 3 mm. The thickness may be up to 20 mm, preferably up to 15 mm, more preferably up to 10 mm. The thickness may thus be in the range of 1 to 20 mm, preferably 2 to 15 mm, more preferably 3 to 10 mm.

It is specified that the first and second flexible radiation-protective sheets are spaced apart from each other by, and attached to, the flexible radiation-transparent sheet. Expressed differently, the flexible radiation-transparent sheet is provided between the first and second flexible radiation-protective sheets.

Preferably the first and second flexible radiation-protective sheets are attached to opposite edges of the flexible radiation-transparent sheet.

This is advantageous in that it provides a low profile, or small thickness, of the radiation-protective covering. Sewing, welding and adhesive are preferred method for attaching the first and second flexible radiation-protective sheets to opposite edges of the flexible radiation-transparent sheet.

Preferably the first and second flexible radiation-protective sheets overlap or overlie the flexible radiation-transparent sheet.

Expressed differently, at least part of one surface of the first and second flexible radiation-protective sheets contacts a surface of the flexible radiation-transparent sheet. This is advantageous in that it simplifies attaching the first and second flexible radiation-protective sheets to the flexible radiation-transparent sheet, e.g. by providing a larger contact surface between the first and second flexible radiation-protective sheets and the flexible radiation-transparent sheet.

Additionally, the flexible radiation-transparent sheet may define a lining for at least part of the radiation-protective covering, e.g. for at least part of the first and second flexible radiation-protective sheets, which may make the radiation-protective covering more comfortable to use for the patient.

This further provides for a simple implementation of the flexible radiation-protective patch as two flexible radiation-protective patch portions. Accordingly, the first and second flexible radiation-protective sheets may be arranged to contact each other along opposing sides of the flexible radiation-protective sheets, and further be arranged to overlie the flexible radiation-transparent sheet. Attaching the flexible radiation-protective sheets to the flexible radiation-transparent sheet using spaced apart attachment lines being parallel with the opposing sides of the flexible radiation-protective sheets defined flexible radiation-protective patch portions integral with the flexible radiation-protective sheets. Preferably the flexible radiation-transparent sheet is substantially rectangular, and at least one of the first and second flexible radiation-protective sheets is rectangular. At least part of the radiation-transparent sheet is extending over the area between the first and second radiation-protective sheets. The flexible radiation-transparent sheet may have a fenestration or aperture as described above.

These shapes are suitable for a simple implementation of the radiation-protective covering.

The radiation-protective covering may be sterile. The radiation-protective covering may be reusable or disposable.

Preferably at least one of the first flexible radiation-protective sheet and the second flexible radiation-protective sheet is shaped as a rectangular hexagon or L-shape.

This provides for obtaining a radiation-protective covering shaped as a rectangular hexagon or L-shape.

Preferably the flexible radiation-transparent material comprises any of a fabric, a mesh, a net, and an impermeable sheet as described above.

These materials are suitable as flexible-radiation transparent materials.

Preferably the flexible radiation-protective material comprises:
a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

These are suitable flexible radiation-protective materials as described above.

The filaments may be arranged in a regular pattern. The filaments may extend in a uniform direction. The uniform direction may be aligned with, or parallel with a folding line or a fold obtained when the flexible radiation-protective patch is folded from the first position to the second position. This increases the suppleness and longevity of the flexible radiation-protective material of the flexible radiation-protective patch.

Additionally, or alternatively, the uniform direction may be aligned with, or parallel with, a length dimension of the radiation-protective covering. Expressed differently, the uniform direction may be aligned with a length dimension of the patient when the radiation-protective covering is in its intended position covering a part of a patient, in particular covering the torso of a patient. This increases the suppleness and longevity of the flexible radiation-protective material of the first and second flexible radiation-protective sheets and may allow the radiation-protective covering to better conform to the shape of the patient.

A second aspect of the proposed technology concerns a use of the radiation-protective covering according to the first aspect of the proposed technology for protecting clinical staff from radiation during a medical intervention comprising the use of radiation.

The medical intervention may comprise a radiologically guided intervention, such as cardiology, or Interventional Radiology (IR). The medical intervention, or medical procedure, may comprise diagnostic and/or surgical procedures. The medical intervention may be a fluoroscopic, or fluoroscopy, assisted intervention.

The use may comprise covering at least a part of a patient with the radiation-protective covering.

The use may further comprise moving the flexible radiation-protective patch from the first position to the second position, and vice versa.

The clinical staff may comprise any personnel present during the medical intervention.

The second aspect of the proposed technology may alternatively be considered a method of protecting clinical staff from radiation during a medical intervention comprising the use of radiation, comprising the steps of:
- providing:
   - the radiation-protective covering according to the first aspect of the proposed technology,
- covering at least a part of a patient with the radiation-protective covering, and optionally,
- moving the flexible radiation-protective patch from the first position to the second position.

### Brief description of the drawings

A more complete understanding of the abovementioned and other features and advantages of the technology proposed herein will be apparent from the following detailed description of preferred embodiments in conjunction with the appended drawings, wherein:
**Figs. 1a** and **1b** show plan and cross-sectional views of an embodiment of a radiation-protective covering with the flexible radiation-protective patch in the first position.
**Figs 1c** and **1d** show plan and cross-sectional views of the embodiment of the radiation-protective covering shown in Figs. 1a and 1b with the flexible radiation-protective patch in the second position.
**Fig. 2a** shows a perspective view of the embodiment of the radiation-protective covering shown in Figs. 1a and 1b placed on a patient positioned on an operating table.
**Fig. 2b** shows a perspective view of the embodiment of the radiation-protective covering shown in Figs. 1a and 1b with a clinical staff member in the process of folding the flexible radiation-protective patch from the first position to the second position.
**Fig. 2c** shows a perspective view of the embodiment of the radiation-protective covering shown in Figs. 1a and 1b with the flexible radiation-protective patch in the second position.
**Figs. 3a** and **3b** show cross-sectional views of second and third embodiments of a radiation-protective covering.

### Detailed description

In the figures and the below description, the same reference numerals are used for the same or related features. One or more' added to a reference number indicates that the feature so designated is a variant of the feature bearing the reference number without the '. A subscript number indicates that the features so references is a further one of the feature bearing the reference number without the subscript number.

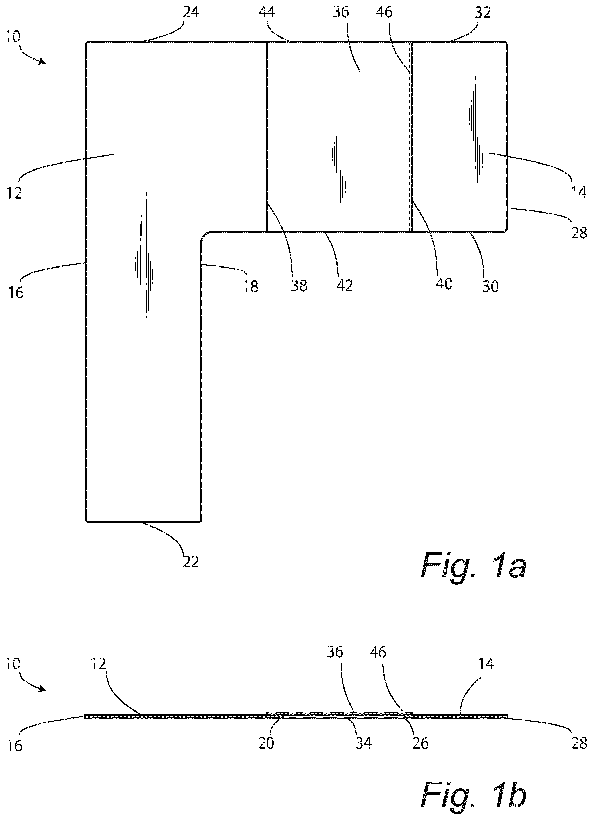

**Figs. 1a** and **1b** show plan and cross-sectional views of an embodiment of a radiation-protective covering 10 comprising a first flexible radiation-protective sheet 12 comprising a flexible radiation-protective material, and a second flexible radiation-protective sheet 14 comprising a flexible radiation-protective material.

Accordingly, the first and second flexible radiation-protective sheets 12, 14 make up the radiation-protective portion.

The first flexible radiation-protective sheet 12 is generally L-shaped having a first long side 16, a second long side divided into first and second long side portions 18 and 20 (shown in Fig. 1b), as well as opposite first and second short sides 22, 24.

The second flexible radiation-protective sheet 14 generally rectangular having opposite first and second long sides 26, 28 (shown in Fig. 1b), as well as opposite first and second short sides 30, 32.

The radiation-protective covering 10 has a width, as defined from the first long side 16 to the second long side 28 of 80 cm. The length of the radiation-protective covering 10, as defined along the direction of the first long side 16, is 90 cm. The perpendicular distance between the first long side 16 and the first long side portion 18 is 20 cm, and the length of the first long side portion 18 is 50 cm.

The second flexible radiation-protective sheet 14 has a width defined as the distance between the first and second long sides 26, 28, i.e. along the first or second short sides 30, 32, of 20 cm, as well as a length defined as the distance between the first and second long sides 30, 32, i.e. along the first or second long side 26, 28, of 30 cm.

The first and second flexible radiation-protective sheets 12, 14 are spaced apart from each other by, and attached to, a flexible radiation-transparent sheet 34 (see Fig. 1b) comprising a flexible radiation-transparent material.

The flexible radiation-transparent sheet 34 thus makes up the radiation-transparent portion.

Accordingly, the radiation-protective covering 10 shown in Fig. 1a comprises a radiation-protective portion comprising first and second flexible radiation-protective sheets 12, 14, and a radiation-transparent portion comprising the flexible radiation-transparent sheet 34.

The flexible radiation-transparent sheet 34 has a length, along the second long side portion 20 and along the first long side 26 of 30 cm and a width between the second long side portion 20 and the first long side 26 of 25 cm.

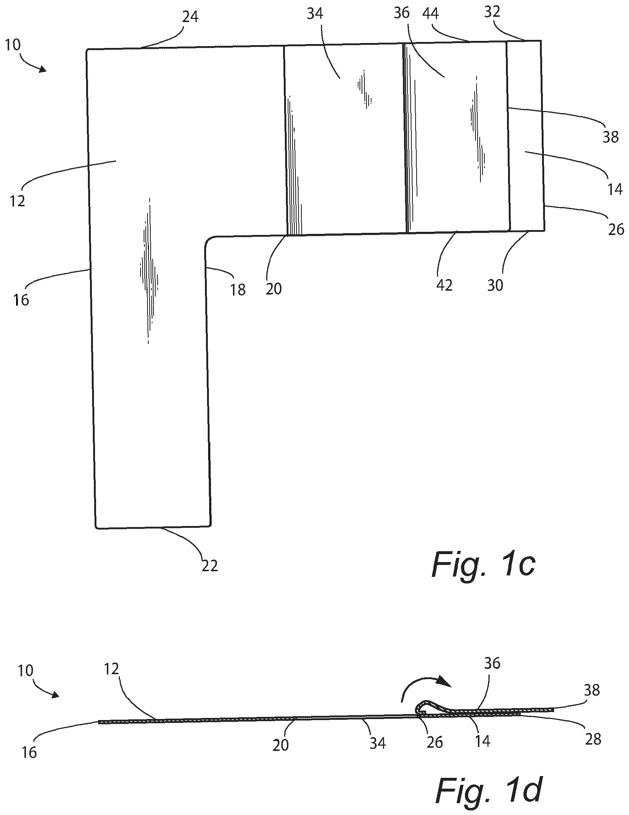

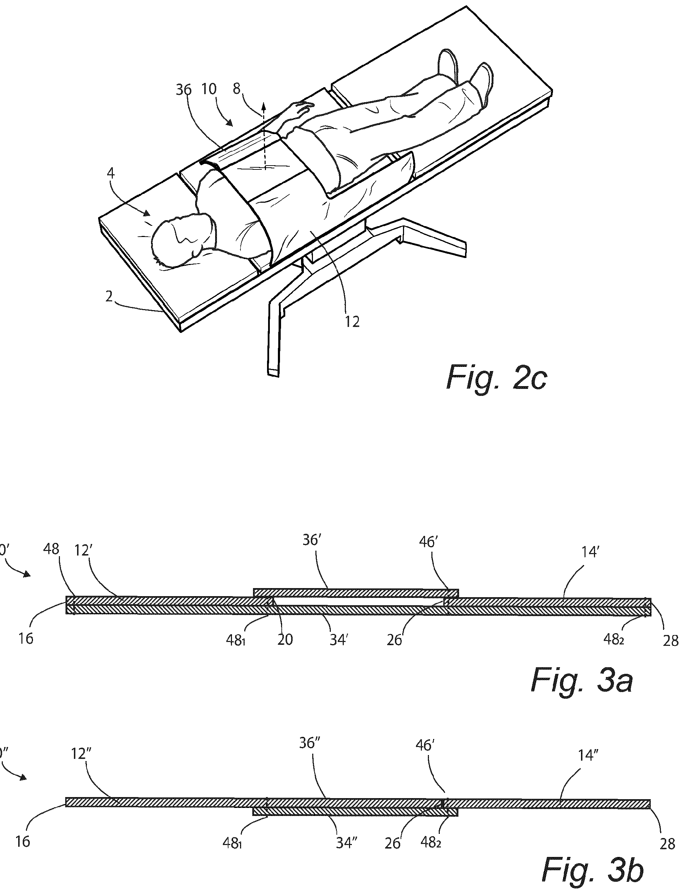

The flexible radiation-transparent sheet 34, shown e.g. in Fig. 1b, 1c, and 2c, is rectangular and is in Fig. 1a covered by a flexible radiation-protective patch 36, comprising a flexible radiation-protective material. The flexible radiation-protective patch 36 has opposing first and second long sides 38, 40 and opposing first and second short sides 42, 44. The length of the flexible radiation-protective patch 36, i.e. distance between first and second short sides 42, 44 is 30 cm and the width, i.e. the distance between the first and second long side 38, 40 is 30 cm.

As seen in Fig. 1a, the radiation-protective patch 36 is attached to the second radiation-protective sheet 14 by stitching 46. As further seen in Fig. 1b the radiation-protective patch overlaps part of the second long side portion 20 on the first radiation-protective sheet 12, as well as part of the first long side 26 on the second radiation-protective sheet 14.

The flexible radiation-protective material of the flexible radiation-protective sheets 12, 14 and of the flexible radiation-protective patch 36 is a permeable fabric that comprises monofilaments arranged in a regular pattern and extending in a uniform direction, as described in WO2014163574A1. Each filament is solid composition that comprises a carrier substance of polyolefin and radiopaque substance of Antimony and Bismuth powder that are uniformly mixed. The fabric is a woven fabric with the filaments forming the weft of the cloth and with a warp of polyester. The flexible radiation-protective material can attenuate X-ray radiation from a radiation source having a tube voltage of between 40 to 150 kV. The flexible radiation-protective material used in the embodiments of the foldable radiation-protective shield shown in the figures has at least 0.50 mm Pb equivalence at any tube voltage in the range 60-110 kV.

In Fig. 1a the flexible radiation-protective patch 36 is shown in a first position in which it covers the flexible radiation-transparent sheet 34 between the first and second radiation-protective sheets 12, 14. In other words, in the first position shown in Fig. 1a the flexible radiation-protective patch prevents radiation from passing through the radiation protective covering 10. Accordingly, as shown in further detail later, the first position of the flexible radiation-protective patch 36 may generally to be used when a clinical procedure is to be performed in the head of a patient, or in a similar position that is distant from the position the clinical staff is positioned. Specifically, in such a procedure the clinical staff is protected from radiation scattered throughout the body of the patient by the second flexible radiation-protective sheet 14, the first flexible radiation-protective sheet 12, and the flexible radiation-protective patch 36 preventing any radiation passing through the flexible radiation-transparent sheet 34 from passing through the radiation protective covering 10.

However, in a second position, the flexible radiation-protective patch 36 is configured to uncover at least part of the radiation-transparent sheet 34 between the first and second radiation-protective sheets 12, 14, as will be described further below.

**Figs 1c and 1d** show plan and cross-sectional views of the embodiment of the radiation-protective covering 10 shown in Figs. 1a and 1b with the flexible radiation-protective patch 36 in the second position. As seen the flexible radiation-protective patch 36 has been folded, at the stitching 46, to lie at least partly on the second flexible radiation-protective sheet 14.

The reason for folding the flexible radiation-protective patch 36 is further explained in the following.

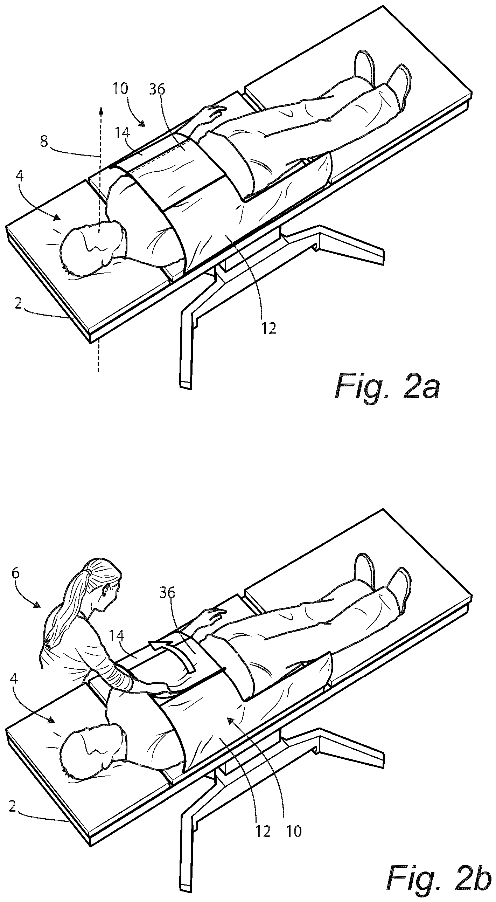

Thus **Fig. 2a** shows a perspective view of the embodiment of the radiation-protective covering 10 shown in Figs. 1a and 1b placed on a patient 4 positioned on an operating table 2. As noted earlier, in a clinical procedure in the head of the patient, radiation 8 may be used to image the structures and anatomy of the head. The amount of scattered radiation emanating from the remainder of the body of the patient is however limited, hence the radiation-protective covering 10, with the flexible radiation-protective patch 36 in the first position covering the flexible radiation-transparent sheet 34, together with suitable protection worn by the clinical staff, is generally sufficient for protecting the clinical staff.

However, during a clinical procedure, the need for imaging the torso and the aorta of the patient may arise. This is due to the fact that a clinical procedure in the head of the patient may involve and/or require that instruments, such as e.g. a catheter, and/or medical devices, such as e.g. a stent, are brought into position in the head via the aorta, i.e. by using e.g. femoral access, and advancing the instrument or device from there through the vasculature including through the aorta to the desired position. This also applies when other positions in the body are to be reached through major passageways, such as major blood vessels, in the body.

Accordingly, by using the radiation-protection covering 10, imaging of the torso and aorta can be easily performed whenever desired by merely moving the flexible radiation-protective patch 36 to the second position. Specifically, even though the part of the patient underlying the flexible radiation-transparent sheet is typically not observable visually, the sheet is transparent to radiation so that the internal structure of the part of the body of the patient can be imaged. Once the need for imaging has passed, e.g. the instrument or device has been safely passed through the area of the torso and aorta of the patient, the flexible radiation-protective patch 36 is moved to the first position to once more provide radiation-protection. In contrast to any prior art radiation-protective shields or blankets placed upon the patient, there is no need to remove the whole radiation-protective covering 10 to gain access to image the torso region and the aorta. Further, a fenestration or aperture may be provided in the flexible radiation-transparent sheet to provide access to the body of the patient.

Further, the flexible radiation-transparent sheet helps maintaining the position of the first and second flexible radiation-protective sheets 12 and 14, and thereby helps maintaining the position of the whole radiation-protective covering 10 on the patient during the clinical procedure.

In line with the above, **Fig. 2b** shows a perspective view of the embodiment of the radiation-protective covering 10 shown in Figs. 1a and 1b with a clinical staff member 6 in the process of folding the flexible radiation-protective patch 36 from the first position to the second position.

Further, **Fig. 2c** shows a perspective view of the embodiment of the radiation-protective covering 10 shown in Figs. 1a and 1b with the flexible radiation-protective patch 36 in the second position, thus allowing the torso and aorta of the patient 4 to be imaged using radiation 8.

**Figs. 3a to 3b** show cross-sectional views of second and third embodiments of a radiation-protective covering.

Fig. 3a thus shows a radiation-protective covering 10' differing from the radiation-protective covering 10 by the flexible radiation-protective sheet 34' extending over the full dimensions of the radiation-protective covering 10', whereby the first and second flexible radiation-protective sheets 12', 14' are attached onto the flexible radiation-protective sheet 34', here using stitching 46', 48, 48₁, 48₂. The flexible radiation-protective flap 36 is also attached to the radiation-protective covering 10' by means of stitching 46'.

The radiation-protective covering 10' is advantageous in that the flexible radiation-transparent sheet 34', which, as discussed herein may be made of various material including fabrics of e.g. cotton, provides a liner function to the radiation-protective covering 10'. In other words, the flexible radiation-transparent sheet 34' provides a lining between the flexible radiation-protective material of the flexible radiation-protective first and second sheets 12', 14'. This may make the radiation-protective covering 10' more comfortable to use for the patient 4. This also decreases the risk of fouling or soiling of the flexible radiation-protective material of the flexible radiation-protective first and second sheets 12', 14', thus increasing longevity and/or cleaning intervals for the radiation-protective covering 10'.

Fig. 3b shows a radiation-protective covering 10'' differing from the radiation-protective covering 10 in that the flexible radiation-protective patch 36'' is integrated as part of the first flexible radiation-protective sheet 12''. As seen, the flexible radiation-transparent sheet 34'' is attached at one side by stitching 48₁ to the first flexible radiation-protective sheet 12" at, or in the proximity of, the center of the flexible radiation-protective sheet 12''. The other side of the flexible radiation-transparent sheet 34'' is attached by stitching 48₂ to the first long side 26 of the second flexible radiation-protective sheet 14''. As the first long side 26 of the second flexible radiation-protective sheet 14'' is not attached to the flexible radiation-protective patch 36'', the flexible radiation-protective patch 36'' may be folded along a fold at stitching 48₁ so as to reveal the flexible radiation-transparent sheet 34".

The radiation-protective covering 10'' is advantageous in that it is of simple construction, requiring only two stitchings.

### Item list

- 2: operating table
- 4: patient
- 6: clinical staff
- 8: radiation
- 10: radiation protective covering
- 12: first flexible radiation-protective sheet
- 14: second flexible radiation-protective sheet
- 16: first long side
- 18: first long side portion
- 20: second long side portion
- 22: first short side
- 24: second short side
- 26: first long side
- 38: second long side
- 30: first short side
- 32: second short side
- 34: flexible radiation-transparent sheet
- 36: flexible radiation-protective patch
- 38: first long side
- 40: second long side
- 42: first short side
- 44: second short side
- 46: stitching
- 48: stitching

## Claims

1. A radiation-protective covering (10) comprising:
- a radiation-protective portion (14) comprising a flexible radiation-protective material, and
- a radiation-transparent portion (34) comprising a flexible radiation-transparent material, and
- a flexible radiation-protective patch (36), comprising a flexible radiation-protective material, configured to:
∘ cover the radiation-transparent portion (34) in a first position, and
∘ uncover at least part of the radiation-transparent portion (34) in a second position.

2. The radiation-protective covering (10) according to claim 1, wherein the flexible radiation-protective patch (36) is configured to be removed from the radiation-protective covering in the second position.

3. The radiation-protective covering (10) according to any preceding claim, wherein the flexible radiation-protective patch (36) is configured to be removably attachable to the radiation-protective covering.

4. The radiation-protective covering (10) according to claim 1, wherein the flexible radiation-protective patch (36) is permanently attached to the radiation-protective covering, preferably wherein the flexible radiation-protective patch (36'') is integrally formed with the radiation-protective portion.

5. The radiation-protective covering (10) according to any of the preceding claims, wherein the flexible radiation-protective patch (36) is configured to be folded from the first position to the second position.

6. The radiation-protective covering (10) according to any preceding claim, wherein the radiation-protective covering is shaped as a rectangular hexagon or L-shape.

7. The radiation-protective covering (10) according to claim 6, wherein the radiation-transparent portion (34) is provided within one of the arms of the rectangular hexagon or L-shape.

8. The radiation-protective covering (10) according to any preceding claim, wherein the radiation-protective portion comprises:
- a first flexible radiation-protective sheet (12), and
- a second flexible radiation-protective sheet (14),
wherein the radiation-transparent portion comprises:
- a flexible radiation-transparent sheet,
and wherein the first and second flexible radiation-protective sheets are spaced apart from each other by, and attached to, the radiation-transparent sheet.

9. The radiation-protective covering (10) according to claim 8, wherein the first and second flexible radiation-protective sheets (12, 14) are attached to opposite edges of the flexible radiation-transparent sheet (34).

10. The radiation-protective covering (10', 10'') according to any of claims 8-9, wherein the first and second flexible radiation-protective sheets (12', 12'', 14', 14'') overlap or overlie the flexible radiation-transparent sheet (34', 34").

11. The radiation-protective covering according to any of the claims 8-10, wherein the first flexible radiation-transparent sheet is rectangular, and wherein at least one of the first and second flexible radiation-protective sheets is rectangular.

12. The radiation-protective covering (10) according to any of the claims 8-11, wherein at least one of the first flexible radiation-protective sheet (12) and the second flexible radiation-protective sheet (14) is shaped as a rectangular hexagon or L-shape.

13. The radiation-protective covering (10) according to any preceding claim, wherein the flexible-radiation transparent material comprises any of a fabric, a mesh, a net, and an impermeable sheet.

14. The radiation-protective covering (10) according to any preceding claim, wherein the flexible radiation-protective material comprises:
a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

15. Use of the radiation-protective covering (10) according to any of the preceding claims, for protecting clinical staff (6) from radiation (8) during a medical intervention comprising the use of radiation.
